Europäisches Patentamt

European Patent Office

Office européen des brevets

(11) Publication number: 0 136 907
A2

# EUROPEAN PATENT APPLICATION

(21) Application number: 84306697.8

(22) Date of filing: 01.10.84

(51) Int. Cl.⁴: C 12 N 15/00

(30) Priority: 03.10.83 US 538730

(43) Date of publication of application:
10.04.85 Bulletin 85/15

(84) Designated Contracting States:
AT BE CH DE FR GB IT LI LU NL SE

(71) Applicant: GENENTECH, INC.
460 Point San Bruno Boulevard
South San Francisco California 94080(US)

(72) Inventor: Heyneker, Herbert Louis
2621 Easton Drive
Burlingame California 94010(US)

(72) Inventor: Lin, Norm Shin Chsiang
698 Crane Avenue
Foster City California 94404(US)

(72) Inventor: Palmer, Donna Thourson
24 East Park Street
Westerville Ohio 43081(US)

(74) Representative: Armitage, Ian Michael et al,
MEWBURN ELLIS & CO. 2/3 Cursitor Street
London EC4A 1BQ(GB)

(54) A xenogeneic expression control system, a method of using it, expression vectors containing it, cells transformed thereby and heterologous proteins produced therefrom.

(57) A portable supplementary control system comprising a promoter/operator operably linked to a gene encoding the desired heterologous protein plus a gene encoding a compatible supplementary repressor protein operably linked to an inducible promotor, thus remedying any deficiency of repressor provided by the host's own genome, so that expression of a gene encoding a heterologous protein is repressed until a favourable point in the growth cycle of the host. The control system offers a method whereby heterologous protein production can be regulated by manipulation of the environment or of the medium.

Croydon Printing Company Ltd.

## A XENOGENEIC EXPRESSION CONTROL SYSTEM, A METHOD OF USING IT, EXPRESSION VECTORS CONTAINING IT, CELLS TRANSFORMED THEREBY AND HETEROLOGOUS PROTEINS PRODUCED THEREFROM

## Background

The invention herein relates to the field of production of heterologous proteins by bacterial hosts using recombinant DNA technology. More specifically, it provides a sensitive control system for regulating the expression of genes encoding heterologous proteins.

Over the past five years, it has been possible to produce a variety of proteins in prokaryotic hosts, which such hosts ordinarily do not produce. These proteins range from those produced by kindred organisms, such as the enzymes conferring tetracycline or ampicillin resistance on its producer, to those from very distant species such as mammalian systems -- proteins such as growth hormone, insulin, and the various interferons. Particularly in the course of producing proteins which are derived from higher organisms, it is advantageous that a system for regulating and controlling the production of the heterologous protein be present in, or imposed on, the host. In the absence of such a regulatory system, production of the foreign protein will typically be coincident with growth of the host organism, and since, in general, the host organism has no metabolic pathways to cope with such protein, it finds the presence of the foreign protein to have a negative impact on growth rate, or even viability. In short, premature production of heterologous proteins by prokaryotic hosts is often extremely toxic.

0406L

Generally, this problem can be managed by postponing the production of the foreign protein to such time in the growth of a cell culture that the cell density is sufficient, and further cell division is not necessary to provide sufficient numbers of host organisms. If, at this time, the synthesis of the heterologous protein is begun, sufficient quantities of it can be produced before the toxic effects are felt to make the process worthwhile. In some cases, foreign proteins can account for upwards of 20 percent of total cell protein, frequently precipitated intracellularly as refractile bodies visible to the eye under a refracting microscope. Against a background of sufficient cell number, acceptable yields of the heterologous protein per liter of cell culture can be achieved in some instances.

A system frequently used to achieve such control has been the trp promoter/operator system which, when operably linked to the gene encoding a desired heterologous protein, can be managed so as to delay (more or less) the production of the heterologous protein to the post growth-phase stage. This system has been described in detail in EPO Application No. 0036776 published September 30, 1981. To achieve regulation with this system, the cells are grown in the presence of tryptophan containing medium so that the trp promoter/ operator is repressed. This occurs because of the production of a repressor protein (trpR) which, in the presence of tryptophan, binds to the trp operator region (Platt, T. in Miller, J.H. and Reznikoff, W.S. [ed.], The Operon, 263-302, Cold Spring Harbor Laboratory (1980)). The gene encoding the repressor protein (the trpR gene) is regulated by the genome of the host organism (Bozogian, G. et al., J. Mol. Biol. 149: 821-825 (1981); Gunsalus, R.P. et al., Proc. Natl. Acad. Sci. (USA), 77: 7117-7121 (1980); and Kelley, R.L. et al., Proc. Natl. Acad. Sci. (USA), 79: 3120-3124 (1982)). Because it is desirable, and often the case, that the trp promoter/operator which controls expression of the heterologous gene is located on a plasmid with high copy number, the quantity of trp repressor protein provided by the hosts' genomic repressor sequence seems inadequate

0406L

to repress the trp promoter/operator completely (Kelly, et al., supra). It is further known that increasing the number of trpR genes in the cell permits a tighter repression of the expression of a gene having multiple copies under trp promoter/operator control (Bozogian, G. et al., supra and Kelly, R.L. et al., supra).

Recently, a portable control system designed for applicability to Gram positive hosts has been devised (US Serial No. 508,388, filed June 27, 1983). However, this system is designed to provide a repressor coding sequence absent in the host, and, further, does not require inducible expression of repressor.

In summary, presently available control systems typified by the above-described trp promoter/operator, while generally helpful in attempts to regulate the timing of heterologous gene expression are not successful in achieving the level of control desirable to maximize the yield of heterologous protein. The present invention offers a system which permits the external regulation of heterologous gene expression with much greater precision so that production can be adequately suppressed during growth phase, and then turned on at will when the cells have reached sufficient growth. This is achieved by providing an operon for the trp repressor xenogeneically under the control of an inducible promoter to supplement that amount of repressor generated endogenously by the hosts' genome.

Summary of the Invention

This invention relates to a system for the control of expression of a gene which encodes a heterologous protein in a prokaryotic host. The system provides a means whereby the promoter/operator which is operably linked to this gene can be effectively suppressed during growth. The system comprises a

0406L

promoter/operator system to regulate the desired gene expression which is capable of being suppressed by the binding of a repressor protein, and a xenogeneic regulable operon for the production of an excess of repressor protein to supplement that available endogeneously. The system, then, comprises the promoter/operator operably linked to the gene encoding the desired heterologous protein plus the gene encoding a compatible supplementary repressor protein operably linked to an inducible promoter, thus remedying any deficiency of repressor provided by the host's own genome. Both of these operon sequences are provided xenogeneically to the prokaryotic host organism. Optionally, high copy numbers of both of them can be obtained.

Thus, in one aspect, the invention relates to the above described control system. In other aspects the invention relates to expression vectors comprising the system, cells or cell cultures transformed with them, and proteins produced by these transformed cells. In a final aspect, the invention relates to a method of enhancing suppression of expression during growth phase of a heterologous gene in a prokaryotic host by culturing cells which are transformed with the control system of the invention under suitable conditions to effect this control.

Brief Description of the Drawings

Figure 1 is a schematic diagram of the construction of an expression plasmid containing the control system of the invention.

Figure 2 represents SDS-PAGE on cellular extracts of cells transformed with pFMB7.

Figure 3 shows the results of SDS-PAGE on cell extracts of cells transformed with pFMB/trpR in the absence of IPTG.

0406L

Figure 4 shows the results of similar procedures to those shown in Figure 3, but where growth occurs in the presence of IPTG.

Figure 5 is a growth curve in minimal medium of cells transformed with pFMB7.

Figure 6 shows the results of SDS-PAGE on extracts of the cell cultures of Figure 5.

Figure 7 shows the growth curve for cells transformed with PFMB/trpR in minimal medium.

Figure 8 shows the results of SDS-PAGE on extracts of the cell cultures of Figure 7.

Detailed Description

A. Definitions

As used herein, DNA sequences which are "operably linked" refers to DNA sequences which are juxtaposed in such a way that their respective functions are mutually dependent. For example, a promoter operably linked to a coding sequence is capable of effecting the expression of the coding sequence. An operator which is operably linked to a promoter is capable, when bound to a repressor protein, of inhibiting the function of the promoter, and when derepressed of permitting it to function as a promoter. Such an operator sequence may overlap the promoter sequence or may lie downstream from it; "operably linked" is independent of such location as long as the functional interrelationship between the two sequences is maintained.

"Compatible supplementary repressor" means a protein which is capable of binding an operator sequence (to which it is compatible) whereby the operator/repressor complex is effective in

0406L

inhibiting the promoter to which the operator is "operably linked"; and which is (or a functional analog of which is) encoded by the host genome it supplements. Thus, a "compatible" repressor has meaning only when referred to a related operator sequence; and "supplementary" only when referred to a particular host.

"Inducible" promoter refers to a promoter which can be "turned on" by derepression in response to simple manipulations such as temperature shifts or addition of compounds to the medium harboring the organisms containing the promoter system, thus providing a voluntary system of expression. A typical example of such an "inducible" control system is the lacUV5 promoter/operator which contains an operator sequence inducible by removing the "lac repressor". Such removal can be induced by adding isopropyl-$\beta$-D-thiogalactoside (IPTG) to the medium; the inducer enters the cells, binds to repressor protein and thus permits expression of the $\beta$-galactosidase gene in untransformed cells or, in cells transformed by recombinant expression vectors of whatever gene is placed in control of the placUV5 promoter. This system is described in detail in U.S. Patent Application 264,306 filed May 18, 1981 and published in EPO publication No. 0067540, Dec. 22, 1982. Another commonly used inducible promoter, which is, however, less sensitive to voluntary control, is the trp promoter/operator, disclosed in U.S. Application Serial No. 133,296 filed March 24, 1980 and published in EPO publication No. 0036776. This promoter is controlled by the binding of trp repressor to the operator, which occurs in the presence of tryptophan. It is inducible by addition of indole acrylic acid (IAA) to the culture medium. The inducer competes with tryptophan for binding to repressor protein, thus inhibiting binding of repressor to the operator. The trp promoter is less finely tuned to voluntary control than the aforementioned lac promoter because a minimal level of derepression is mandated by the required presence of tryptophan needed for protein synthesis.

0406L

"Portable" control system means that the system is capable of being ligated operably to a desired gene sequence, and capable of controlling expression in prokaryotic hosts in general -- not just in the prokaryotic host of its origin. Such potential hosts include bacteria spanning the entire taxonomic scope of prokaryotes.

"Control system" means a DNA sequence or sequences which enable the production of a desired protein to be regulated at will. A control system would inevitably include the promoter which regulates transcription of the desired gene plus some mechanism for turning that promoter off and on. In the commonly encountered fashion of prokaryotes, this "on/off" regulation is accomplished by means of a repressor protein which is bound to an operator region of the operon. As it relates to the invention, the control system will also include a means for regulating the levels of repressor.

"Heterologous protein" most typically refers to protein which is ordinarily not produced by the host cell. However, it includes any protein which is produced in that host by virtue of the inclusion of DNA sequences which are foreign to the organism, even if the host has its own sequences enabling production of the same amino acid sequence. In short, a heterologous protein is the product of xenogeneic transformation into the host.

"Xenogeneic" refers to DNA sequences which are transformed into a host cell. They may or may not be identical to or similar to sequences which are already contained in the host cell's genome or episomally therein. They are "xenogeneic" by virtue of their being introduced by transformation of the host.

There are two sets of terms which are used herein as general descriptions and without intent as to limitation as to their precise meanings. One set is "peptide, polypeptide, and protein". Each of these terms refers to an amino acid sequence which may or may not have associated acetylation, phosphorylation, glycosylation,

0406L

lipids, and the like; may occur either in neutral form or as a salt according to its environment; and may be altered by environmental factors, such as, for example, reduction of disulfide linkages, or oxidation or sulfitolysis of sulfhydryl groups. Further, the size distinctions arbitrarily made between peptide and protein in some contexts are not observed here. All of these terms refer to an amino acid sequence of any length, in any ionic state, and any state of modification as set forth above. Specifically named proteins and peptides are also defined so as to include this scope.

Similarly, unless evident from the context, no distinction is made between "cells" and "cell cultures". Transformation of "cells" is ordinarily done in the context of a "cell culture", purifications are ordinarily made originally from a "cell culture" by harvesting the "cells" or by recovering the desired material from the supernatant after the "cells" are removed. In any case, where a distinction is not clear from the context, "cells" and "cell cultures" are meant to cover both.

B. General Description

The portable control system of the present invention basically comprises a first DNA sequence comprising a promoter/operator sequence operably linked to the gene for a desired heterologous protein and a second DNA sequence which encodes a repressor compatible with that promoter/operator and which is itself regulated by its own inducible promoter. The repressor supplements an endogenous genomic repressor. Both of these DNA sequences comprising the elements of the portable control system are capable of being transformed effectively into a prokaryotic host.

In a typical construction of the control sequence, a prokaryote-compatible plasmid, such as, for example, pBR322 and derivatives thereof, or pRSF1010, is modified using suitable

0406L

restriction enzymes to insert the elements of the control system and the desired gene. The promoter/operator sequence which will be operably linked to the desired gene is inserted either separately or together with the desired gene by use of the conventional restriction enzymes and appropriate end tailoring through blunt ending and/or linkers as is understood in the art. The operon for the compatible repressor is similarly inserted in this plasmid. The spatial relationship between these two elements of the control system on the plasmid does not matter.

Suitable promoter/operators include, for example, the trp promoter (supra), the lac promoter (supra), the tac promoter, and the λpL promoter (Bernard, H., et al., Gene, 5: 59 (1979)), preferably the trp promoter. Suitable embodiments of the second element of the control system are the coding sequences for compatible repressors for each of the aforementioned promoter/operator sequences each operably linked to an inducible promoter, preferably the plac UV5 promoter. However, other, for example, temperature sensitive inducible promoters are available, such as λpL described in Bernard, et al. (supra).

Alternative constructions permit placing the first and second elements of the control system on separate plasmids such that a host cell would then be cotransformed with a mixture containing both of them. Alternatively, a transformation vector or vectors bearing one or both of the elements can be modified by ligating into the plasmid sequences similar to those of the bacterial genome, such that the transformed host will integrate the material of the plasmid into its chromosomal DNA.

Any DNA sequence which encodes a desired protein can be used as the gene whose expression is to be regulated. Many examples of such sequences which have been used in recombinant technology and which are obtainable from alternate expresssion plasmids are available. For example, the genes for human insulin, α-, β-, and γ-interferons, animal interferons, human and animal growth hormones,

0406L

and a number of antigenic peptides such as FMD polypeptides, and influenza hemaglutinin have been constructed in this way. Also available are the coding sequences for tissue plasminogen activator, urokinase and lymphokines such as interleukin 2 (IL-2). The control system is suitable for these sequences, already available in the art, as well as for sequences not presently available which encode a desired protein, and which may become available in the future.

In employing the system, the host organism is transformed by the vector or vectors containing the elements of the system and grown under conditions which favor the expression of the gene encoding the supplemental compatible repressor. These conditions may not be the same as conditions which favor the expression of the same repressor coding sequences residing in the host cell chromosome. However, expression of the host chromosomal repressor codons, which would require additional environmental regulation, may not be necessary as sufficient repressor may be provided by the xenogeneic sequences. It will be noted that in most instances the promoter controlling the initiation of transcription of the genomic repressor and of the control system repressor sequences will not be the same, and hence similar conditions will not succeed in stimulating synthesis of the repressor in both. For example, in the example set forth below, the genomic trp repressor sequence is under control of its own promoter, which is repressed by a tryptophan/trp repressor complex. On the other hand, the trp repressor provided in the control system is under the control of the lac promoter which is activated (derepressed) in the presence of isopropyl-β-D-thiogalactoside (IPTG). If this is the repressor used, however, tryptophan will in any event have to be added to the medium since it is required for repressor binding to the trp operator.

In the typical use of the control system of the invention, transformed cells are initially grown in the presence of a chemical inducer or other environmental factor which derepresses compatible supplementary repressor production. When the cells have grown to a suitable cell density, for example about $O.D._{550}$ of 10-100, the

0406L

factors which induce production of repressor are altered so that repressor production by the xenogeneic system stops and the promoter/operator, thus freed of these supplemental amounts of repressor protein, permits the synthesis of the desired polypeptide. Depending on the inducible promoter used for expression of repressor and on the promoter/operator for the heterologous gene, this alteration of conditions can be accomplished by, for example, adding galactose, removing IPTG, raising or lowering the temperature, adding trp inhibitors or other suitable manipulations appropriate to the promoter in question. If the trp promoter/operator is the controlling element for the desired gene, it is also desirable (but not part of this invention) to add an inhibitor of the repressor binding, such as, in that case, indole acrylic acid (IAA) which interferes with the binding even of repressor protein that has already been synthesized or which is genomically synthesized. Thus, derepression of the promoter/ operator controlling the desired gene is effected by turning off the xenogeneic repressor synthesis, optionally enhancing this effect by interfering with the repressor binding.

The following examples are intended to illustrate the invention but not to limit its scope. The particular promoter/operator and promoter systems used are among the most common, but any promoter/operator which is regulated by a repressor protein which is, or an analog of which is, encoded genomically by the host and any promoter which is inducible in response to environmental factors can be used in the constructions. Similarly, while a one plasmid construction is illustrated, the aforementioned alternative approaches can likewise be used.

### C. Examples

#### C.1 Methods of Vector Construction

Construction of suitable vectors containing the desired coding and control sequences employs standard ligation techniques

0406L

well understood in the art. Isolated plasmids or DNA fragments are cleaved, tailored, and religated in the form desired to form the plasmids required. The methods employed are not dependent on the DNA source, or intended host.

Cleavage is performed by treating with restriction enzyme (or enyzmes) in suitable buffer. In general, about 1 µg plasmid or DNA fragments is used with about 1 unit of enzyme in about 20 µl of buffer solution. (Appropriate buffers and substrate amounts for particular restriction enzymes are specified by the manufacturer.) Incubation times of about 1 hour at 37°C are workable. After incubations, protein is removed by extraction with phenol and chloroform, and the nucleic acid is recovered from the aqueous fraction by precipitation with ethanol.

If blunt ends are required, the preparation is treated for 30 minutes at 37° with 10 units of E. coli DNA Polymerase I (Klenow), phenol-chloroform extracted, and ethanol precipitated.

Size separation of the cleaved fragments is performed using 6 percent polyacrylamide gel described by Goeddel, D., et al, Nucleic Acids Res., 8: 4057 (1980) incorporated herein by reference.

For ligation, approximately equimolar amounts of the desired components, suitably end tailored to provide correct matching are treated with about 10 units T4 DNA ligase per 0.5 µg DNA for 2 hr. at 22°C or 16 hr. at 12°C.

In the examples described below correct ligations for plasmid construction are confirmed by transforming E. coli K12 strain 294 (ATCC 31446) or other suitable host strain with the ligation mixture. Successful transformants were selected by ampicillin or tetracycline resistance or combination thereof, or using other markers, depending on the mode of plasmid construction. Plasmids from the transformants are then prepared, analyzed by

0406L

-13-

restriction nuclease digests and/or sequenced by the method of Messing, et al, Nucleic Acids Res., 9:309 (1981) or by the method of Maxam, et al, Methods in Enzymology, 65:499 (1980).

Transformations may be carried out employing the method of Cohen, S.W., et al., Proc. Nat'l. Acad. Sci. (USA) 69: 2110 (1972).

C.2 Construction of Vectors Containing the Control System of the Invention

Using the method set forth in paragraph C.1, a controlled expression vector for a typical protein, an FMD antigen, which includes the control system of the invention, pFMB/TrpR, was constructed from three plasmids available in the art:

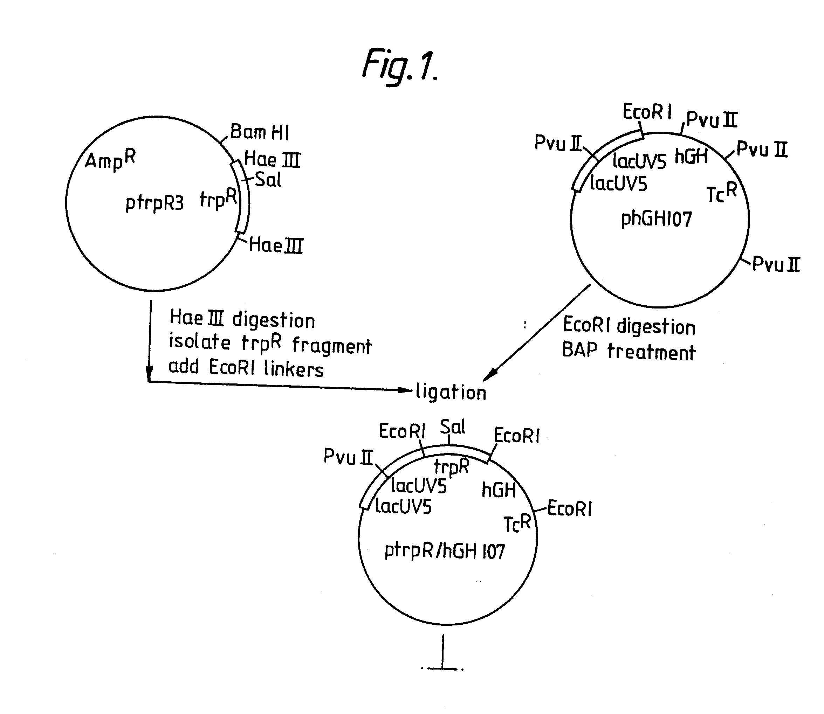

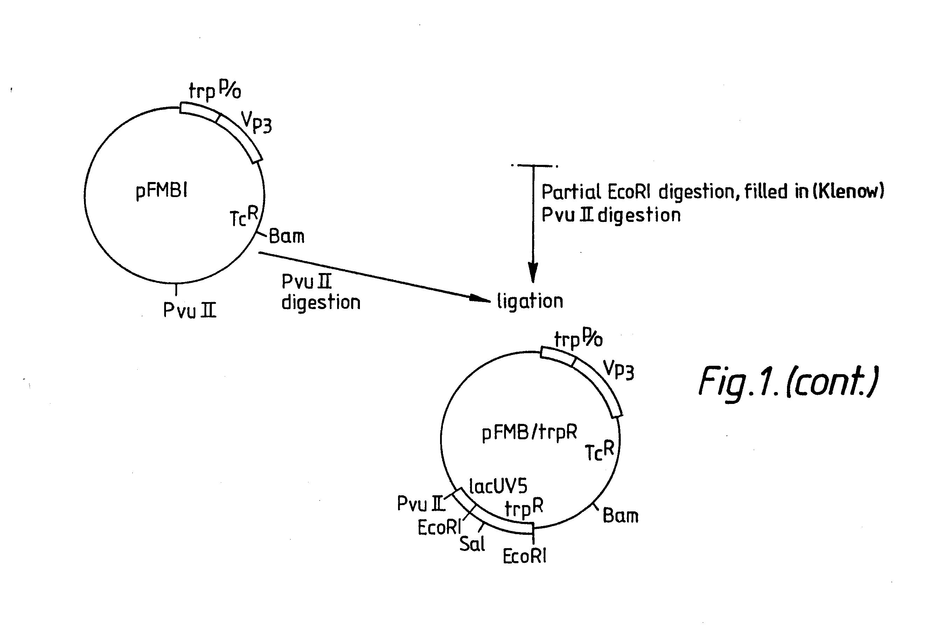

phGH107, described in EPO Publication No. 022242, published January 14, 1981, was used as a source for the lac inducible promoter. pTrpR3, described in Roeder, W. et al., Molecular Genetics, 176: 361 (1979) was used as the source of the coding sequence for trp repressor. pFMB1, described in EPO Publication No. 0068693 published January 5, 1983, was used as the source of the coding sequence for the FMD antigen derived from strain A24. The plan of construction is shown in Figure 1.

To obtain the trp repressor sequence, ptrpR3, was treated with HaeIII, and the 334 base pair fragment isolated on 6 percent acrylamide gel. The isolated fragments were ligated with 16-mer EcoR1 linkers having the sequence: 5'CCATAGAATTCTATGG. To obtain vector backbone and the lac promoter, phGH107 was first digested with EcoR1, and treated with bacterial alkaline protease. The large vector fragment containing the lacUV5 promoter was then ligated to the tailored trpR fragment using T4 ligase, and the ligation mixture transformed into E. coli. Plasmid DNA from successful transformants was isolated and the presence of the desired plasmid, designated ptrpR/hGH 107, confirmed by miniscreen (see Maxam, supra).

0406L

ptrpR/hGH107 was partially digested with EcoRl, blunt ended using Klenow, treated with PvuII to provide the lac promoter/trp repressor operon (the 530 b.p. fragment). Partial PvuII digestion of pFMB1 and isolation of the vector fragment on 6 percent polyacrylamide provides the expression vector backbone containing the FMB coding sequence under control of the trp promoter. The ptrpR/hGH107 fragment was mixed with the pFMB1 PvuII digest and ligated with T4 ligase. The ligation mixture was then used to transform E. coli strain 294, and successful transformants used as a source of plasmid DNA. The resulting plasmids, pFMB/trpR were verified by miniscreen, and for orientation of the insert by AvaI-PvuII digestion, and used in the procedures set forth in paragraph C.3 below to test the ability of the control system to supress premature FMB production. pFMB7 was used as a control plasmid to compare expression patterns without this control system.

C.3 Comparison of FMD-Antigenic Protein Production by Cultures with and without Xenogeneic trp R Sequences.

Fermentations were carried out using cultures transformed with plasmids as described in C.2, pFMB/trpR and pFMB1, containing the FMD antigen gene under the control of the trp promoter with and without the trpR gene under placUV5 control. Determinations were made at various cell densities of the content of FMD antigenic protein by removing samples, spinning down the cells, lysing the cells by sonication, and subjecting the lysed sonicate to SDS-PAGE.

The cultures were all grown in LB media + 5μg/ml tetracycline HCl, with and without specified concentrations between 0.1 and 0.2 mM IPTG. After an 8 hour incubation, this inoculum was transferred to a fermenter containing M9 minimal salts plus other supplements such as casamino acids and 50 μg/ml tryptophan.

The results, illustrated below, showed that higher cell densities were obtainable in the presence of trpR gene in the transforming vectors and with IPTG inducer in the medium to derepress

0406L

expression of trpR and that in the presence of this control system, production of the FMD antigenic protein was delayed.

In all of the figures illustrating SDS–PAGE in this Example, the (-) lane represents extracts of control cells transformed with vectors lacking the FMD virus antigen gene, and the LMW band represents size standards.

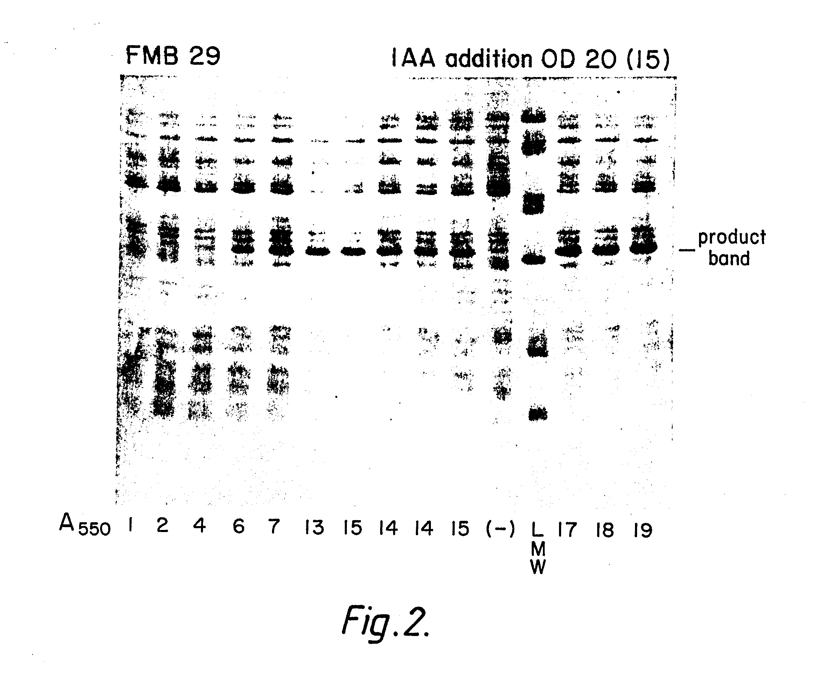

Figures 2–4 show the SDS–PAGE analysis at various values of $OD_{550}$ of lysed cultures grown in M9 salts plus supplements.

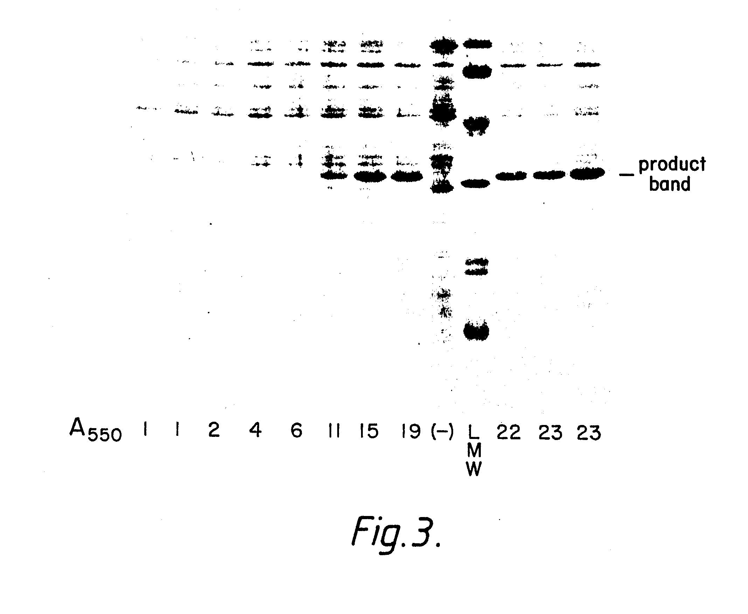

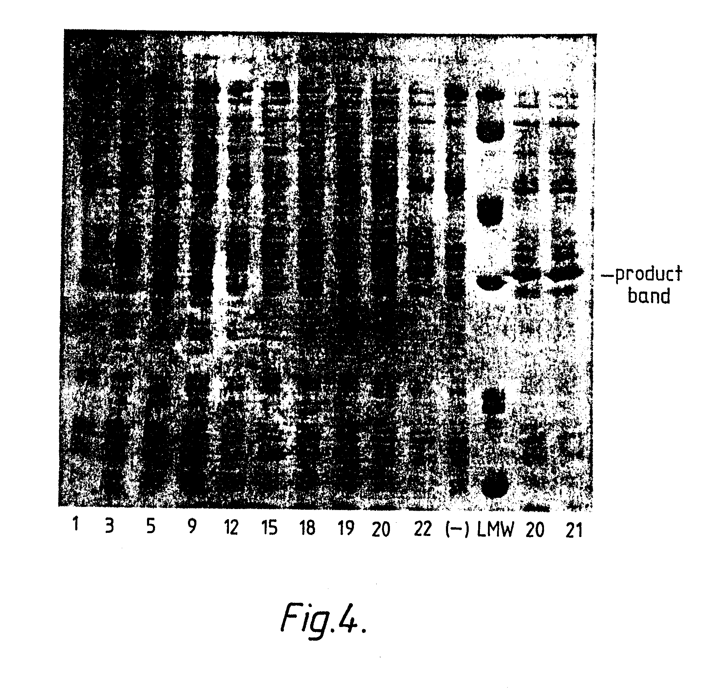

Figure 2 shows that the production of FMD–viral antigen in cells lacking the control system of the invention is detectable beginning at $OD_{550} = 6$ and continues at roughly the same level through $OD_{550} = 19$. On the other hand, Figure 3 which represents a similar SDS–PAGE run on cells containing the entire portable control system (but without IPTG) shows no FMD antigen protein produced until $OD_{550} = 11$. In cells of Figure 4, which contain both the control system and the IPTG inducer, thus taking advantage of the inducible repressor control, product is not formed in detectable quantity until $OD_{550} = 19$.

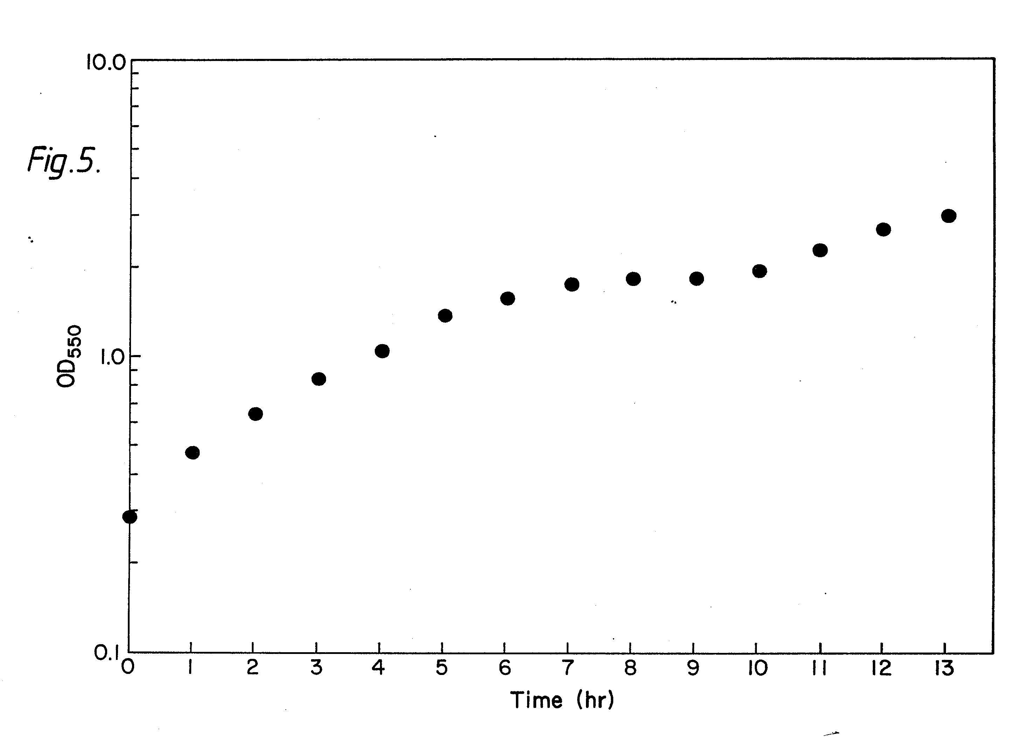

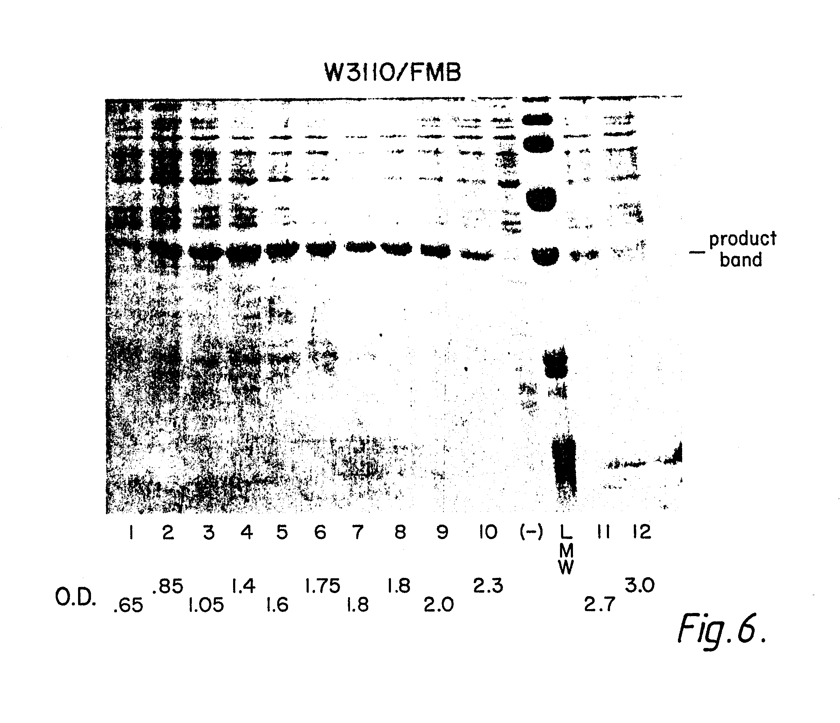

Figures 5–8 give the results of similar experiments using a slightly different medium in the fermenter, i.e., minimal medium plus tryptophan. Figure 5 indicates the growth of cells containing no xenogeneic trpR repressor coding sequences (pFMB1 transformants) is dramatically inhibited so that approximately OD = 3 is the extent of growth after 13 hours. Figure 6 shows the SDS–PAGE of this cell culture, which indicates that product is produced during all this slow growth phase.

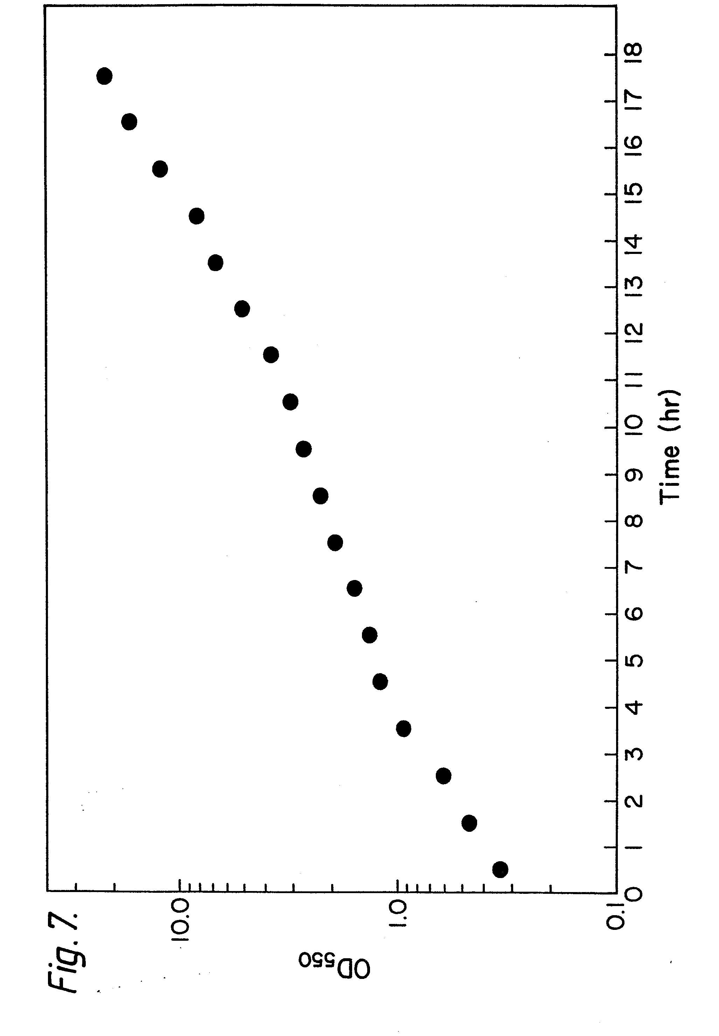

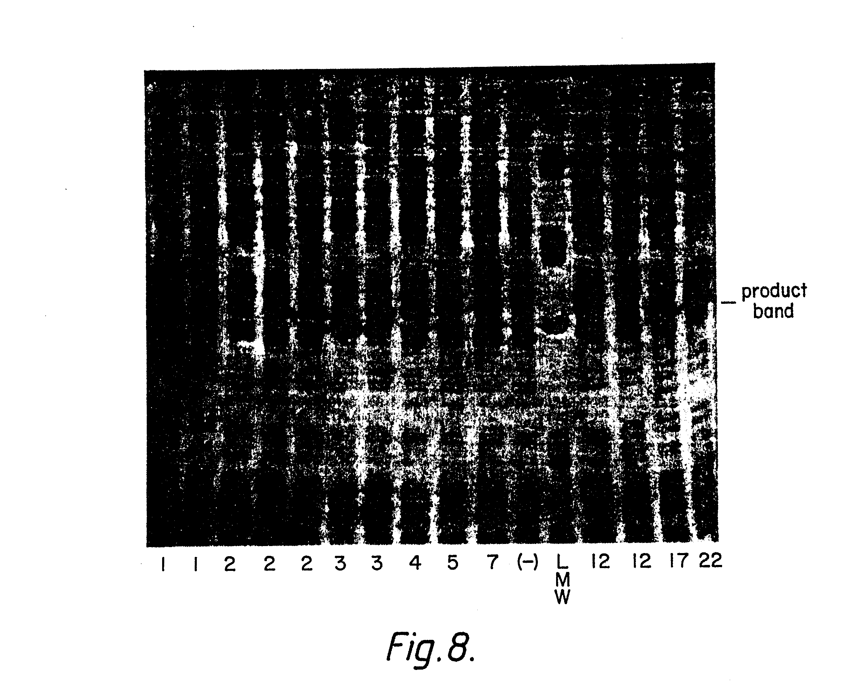

Figures 7 and 8 show the corresponding results for cells transformed with the complete control system and FMD antigenic protein encoding operon (pFMB/trpR transformants) without IPTG. In this case an OD of 22 was achievable after 17 hours and no product band appears in the SDS gel (Figure 8) until OD = 17.

Claims

1. A control system useful in suppressing the expression of a gene encoding a protein heterologous to a prokaryotic host which system comprises:

a first DNA sequence which comprises a promoter/operator operably linked to the gene encoding said protein; and

a second DNA sequence which comprises an inducible promoter operably linked to the gene encoding a repressor protein compatible with the promoter/operator, and supplementary to the host;

wherein the first DNA sequence and second DNA sequence are xenogeneic to the host.

2. The control system of Claim 1 wherein the first DNA sequence comprises the trp promoter/operator.

3. The control system of Claim 1 wherein the second DNA sequence comprises the lac promoter.

4. The control system of Claim 2 wherein the second DNA sequence comprises the lac promoter operably linked to the trpR gene.

5. The control system of Claim 1 wherein the host is a strain of E. coli.

6. The control system of Claim 1 wherein the heterologous protein is selected from the group consisting of FMD antigenic protein, a mammalian growth hormone, a mammalian interferon, human tissue plasminogen activator, urokinase and IL-2.

7. An expression vector effective in prokaryotic host cells in expressing a gene encoding a desired protein heterologous to said host, which comprises the control system of Claim 1.

0406L

8. Cells or a cell culture transformed with the vector of Claim 7.

9. A protein produced by expression of the gene encoding a heterologous protein disposed in the vector of Claim 7.

10. A method of controlling the expression of a gene encoding a heterologous protein, which method comprises culturing the cells of Claim 8.

-11. A method of controlling the expression of a gene encoding a desired heterologous protein, which method comprises culturing cells transformed with an expression vector for said gene which vector comprises

a first DNA sequence which comprises a promoter/operator operably linked to the gene encoding said protein; and

a second DNA sequence which comprises an inducible promoter operably linked to the gene encoding a repressor protein compatible with the promoter/operator, and supplementary to the host;

wherein the first DNA sequence and second DNA sequence are xenogeneic to the host.

Fig.1.

Fig. 1. (cont.)

0136907

FMB 29          IAA addition OD 20 (I5)

product band

A₅₅₀  I  2  4  6  7  I3  I5  I4  I4  I5  (−)  L M W  I7  I8  I9

*Fig.2.*

0136907

3/9

A$_{550}$  1  1  2  4  6  11  15  19  (−)  L M W  22  23  23

product band

4/9

0136907

*Fig.3.*

—product band

1  3  5  9  12  15  18  19  20  22  (—)  LMW  20  21

Fig.4.

0136907

Fig.5.

# W3110/FMB

product band

| Lane | 1 | 2 | 3 | 4 | 5 | 6 | 7 | 8 | 9 | 10 | (−) | LMW | 11 | 12 |

O.D. .65 .85 1.05 1.4 1.6 1.75 1.8 1.8 2.0 2.3 2.7 3.0

*Fig.6.*

Fig. 7.

OD$_{550}$

Time (hr)

0136907

I I 2 2 2 3 3 4 5 7 (–) L M W 12 12 17 22

— product band

Fig.8.

0136907